# EUROPEAN PATENT SPECIFICATION

(11) **EP 3 205 115 B1**
(45) Date of publication and mention of the grant of the patent: **27.05.2020**
(21) Application number: 16810440.4
(22) Date of filing: 22.11.2016
(51) Int. Cl.: H04R 1/10, G10K 11/178

(54) **WIRELESS NOISE-CANCELLING EARPLUG**
DRAHTLOSER OHRSTÖPSEL MIT RAUSCHUNTERDRÜCKUNG
OREILLETTE ANTIBRUIT SANS FIL

(30) Priority: 24.11.2015 FI 20155870
(43) Date of publication of application: 16.08.2017
(73) Proprietor: QOn OY, 90440 Kempele (FI)
(72) Inventor: KYLLÖNEN, Janne, 90810 Kiviniemi (FI); NISULA, Matti, 90520 Oulu (FI); SARLUND, Pekka, 90480 Hailuoto (FI)
(74) Representative: Papula Oy
(86) International application number: PCT/FI2016/050818
(87) International publication number: WO 2017/089649

(56) References cited:
- WO-A1-2006/003618
- GB-A- 2 453 434
- US-A- 4 455 675
- US-A- 5 937 070
- US-A1- 2008 107 287
- US-A1- 2011 223 864
- US-A1- 2015 139 474
- Doppler Labs: "Here Active Listening - Change The Way You Hear The World", , 1 July 2015 (2015-07-01), XP055345207, Retrieved from the Internet: URL:https://www.kickstarter.com/projects/d opplerlabs/here-active-listening-change-th e-way-you-hear-the/description [retrieved on 2017-02-13]
- G.R.A.S. Sound & Vibration A/s: "G.R.A.S. 45BC-1 KEMAR Head & Torso with Mouth Simulator for Headset Test, 2-Ch LEMO", , 7 October 2015 (2015-10-07), XP055505887, Retrieved from the Internet: URL:https://web.archive.org/web/2015100720 1404/http://www.gras.dk:80/45bc-1.html [retrieved on 2018-09-10]
- "Electroacoustics - Simulators of human head and ear - Part 7: Head and torso simulator for acoustic measurement of hearing aids", IEC TS 60318-7:2011, IEC, 3, RUE DE VAREMBÉ, PO BOX 131, CH-1211 GENEVA 20, SWITZERLAND, 24 February 2011 (2011-02-24), pages 1-35, XP082001568, [retrieved on 2011-02-24]
- V.R. Algazi ET AL: "The CIPIC HRTF database", Applicationis of Signal Processing to Audio and Acoustics, 2001 IEEE W orkshop on the Oct. 21-24, 2001, 1 January 2001 (2001-01-01), pages 99-102, XP055541502, DOI: 10.1109/ASPAA.2001.969552 ISBN: 978-0-7803-7126-2

## Description

### TECHNICAL FIELD

The present disclosure relates to earplugs. Particularly, the present disclosure relates to wireless noise cancelling earplugs.

### BACKGROUND

Earplugs are used in various situations where protection against or alleviation of outside noise is required. In many situations there is a need for earplugs that are small, easy to use and affordable and, at the same time, effective in cancelling noises of different frequencies.

For example, cabin noise in airplanes is a phenomenon of fairly low frequencies where the peak is around 70 Hz, and the peak cabin noise at this frequency can be as high as 90 dB. This can be especially stressful and annoying when trying to sleep during a flight, or when simply trying to relax in a noisy environment.

Also music, speech or other noise coming through walls of an apartment may create a stressful environment where attenuation of the unwanted noise is needed, especially during night time when the person suffering from the noise is trying to sleep or rest. The attenuation of such noise of fairly low frequencies would be desired.

The audio environment inside a motorcycle helmet of a motorist whilst riding has similar conditions, as well, which makes listening to radio and/or music while riding uncomfortable, as unwanted noise frequencies interfere or disrupt those of the desired audio input.

Earplugs with passive noise reduction are typically relatively cheap, simple structures made of foam plastic, memory foam, silicone, coated wax or other such malleable materials suitable for moulding in one's hands and inserting into one's ear canal where they subsequently expand to fill out the ear canal to passively reduce the noise reaching the audio organs. They do not offer very good protection against noise in the above-described situations. While their noise reduction capability increases with noise frequency, it is not particularly good at low frequencies.

On the other hand, active noise cancellation (ANC) works well at low frequencies, utilising a microphone for detecting noise, a Digital Signal Processor (DSP) or other signal processing equipment such as an analogue filter for processing the noise, and a speaker for producing antinoise cancelling the noise to be controlled. Commonly, ANC solutions utilise feedback or feed-forward topologies, or a combination of the two. In most embodiments ANC devices work well for noise with frequency 1 kHz or less. In addition, the present solutions for active noise cancellation can be relatively expensive and/or so cumbersome in their size and design that their use in the above-described situations is not feasible. Typically, ANC solutions are utilised in earplugs or headphones designed particularly for listening to music, and they are not suitable for sleeping or resting one's head against a pillow or a headrest.

Large earphones, or earplugs that extend from the ear beyond the tragus, antitragus and antihelix of the ear can well be worn when the user is standing, sitting or lying on his/her back. However, such earphones or earplugs are uncomfortable to wear when lying sideways or wearing head protection such as a helmet.

Different earplugs for sound enhancement, sound quality enhancement or noise attenuation are presented for example in US 2015/139474 A1, US 2011/223864 A1, US 2008/107287 A1, WO 2006/003618 A1 and GB 2453434 A, as well as on Doppler Labs "Kickstarter" pages (hppts://www.kickstarter.com/projects/dopplerlabs/her e-active-listening-change-the-way-you-hear-the/description).

### SUMMARY

There is provided wireless noise-cancelling earplug comprising a housing within which at least an active noise cancellation ANC circuit for producing anti-noise, a speaker for emitting the anti-noise as a sound wave, and a battery for powering at least the ANC circuit are arranged; the earplug further comprising an audio cavity for guiding the sound wave from the speaker out of the earplug, at least one microphone for measuring ambient noise and feeding the measured ambient noise to the ANC circuit, and a passive noise reduction unit for blocking ambient noise, the earplug and the housing as viewed from one side is L-shaped comprising a stem portion of the L-shaped housing along a first axis and bar portion of the L-shaped housing along a second axis, wherein the stem portion has a length of 25 mm or less; the bar portion has a length of 23 mm or less; an inner angle between the first axis and the second axis of the L-shaped housing is 85 to 120 degrees; a thickness L3 of the earplug on the part of the stem portion that does not intersect with the bar portion is 5 to 9 mm; and the at least one microphone is arranged inside the audio cavity.

In one embodiment there is provided a noise cancelling earplug employing both passive and active noise cancellation. In an embodiment the earplugs are implemented so as to be affordable, and with noise cancellation that fits inside the user's ear. In an embodiment the earplugs requires no external power source while in use, but have a battery as power source that can be charged from time to time. In an embodiment the earplug reduces both high and low frequency noise, and is easy to use, and therefore overcomes or at least reduces the problems associated with the prior solutions as discussed above.

In one embodiment there is provided a convenient and user-friendly earplug comprising ANC, in which the number of user-operable components, such as switches or other controlling equipment, is avoided or minimised. In one embodiment, when the earplug is in use it has no disturbing or obtrusive power lines or cords. In an embodiment the earplug has its own power source in form of a rechargeable battery. Such a battery may be charged by connecting the earplug to a charger either via connectors, or it may be charged wirelessly, e.g. using inductive charging.

In an embodiment, there is provided an earplug having a size small enough to fit into the user's ear entirely. The entire earplug, apart from the bud part inserted into the ear canal, may be fitted into the concha and the intertragical notch of the user's outer ear, so that the earplug becomes situated between the tragus, the antitragus and the antihelix. This makes leaning against, for example, an airplane cabin seat headrest or a pillow more comfortable.

Herein the term 'earplug' means a device which is meant to be inserted at least partially into the ear canal of a user's ear to reduce noise. Earplugs are normally used as a pair, for both ears of the user.

Herein the term 'housing' means the outer casing or body of the earplug, which housing encompasses, protects and covers all pieces or some of the pieces of the electronic equipment of the earplug. In an embodiment the 'housing' may also provide the earplug its outer shape and design. However, in some embodiments a separate cover may be provided on top of the housing where the cover provides the outer shape and design of the earplug.

Herein the term 'sealing bud' means a part of an earplug which is meant to be inserted into the ear canal of the user. In an embodiment the sealing bud may act as passive noise reduction equipment. In one embodiment the sealing bud may be separate from the housing. In another embodiment the sealing bud may be an integrated part of the housing.

Herein the term 'Digital Signal Processor' or 'DSP' means a microprocessor that is used for digital signal processing.

Herein the term 'active noise cancellation' or 'ANC' means active cancellation of certain frequencies of noise by producing antinoise or counteracting noise. The ANC may be implemented electronically.

In one embodiment high frequency noise includes noise of a frequency higher than 1 kHz. In another embodiment high frequency noise includes noise of a frequency higher than 2 kHz. In an embodiment high frequency noise may extend to 10 kHz and even above that.

Correspondingly low frequency noise includes in one embodiment noise of frequency under 1 kHz, and in another embodiment noise of frequency 2 kHz and less.

In one embodiment, the speaker is arranged into the intersection of the first axis and the second axis; and the housing comprises along the first axis at least the speaker and the battery; and along the second axis at least the speaker and the at least one microphone.

In one embodiment, the earplug comprises at one end of the housing along the first axis a sealing bud, wherein the length of the bar portion includes the dimension of the housing and the sealing bud along the first axis.

In one embodiment, the audio cavity comprises a first part arranged inside the housing along the second axis, and a second part arranged to protrude from the housing along a third axis, so that an inner angle between the first axis and the third axis is 85 - 110 degrees.

In one embodiment, the housing (12) comprises a first cylindrical part arranged along the first axis so that the normal axis of the first cylindrical part is substantially perpendicular to the first axis; a second cylindrical part whose axis is the second axis; a first surface which is the top circular plane surface of the first cylindrical part and which is parallel to the first axis and opposite the audio cavity; and a second surface, which is the bottom circular plane surface of the first cylindrical part and which is parallel to the second axis and on the same side as the audio cavity.

In one embodiment, the battery is arranged within the housing inside the first cylindrical part of the housing.

In one embodiment, at least the active noise cancellation ANC circuit, the speaker and the first part of the audio cavity are arranged within the second cylindrical part.

In one embodiment, at least one microphone is arranged outside the audio cavity and the housing.

In one embodiment, the width of the earplug on the part of the stem portion that does not intersect with the bar portion is 13 - 16 mm.

In one embodiment, the housing comprises charging sockets arranged on the first surface of the housing on the stem portion.

In one embodiment, the ANC circuit is configured to cancel noise at frequencies of 1 kHz or less.

In one embodiment, the earplug is intended to be placed into the ear so that the bar portion is placed in direction of and at least partly inside the ear canal, and the stem portion extends from the ear canal to the concha.

In one embodiment, the earplug is intended to fit in its entirety within a space inside the outer ear so that the passive noise reduction part fits into the outermost part of the ear canal and the housing fits into a space comprising the concha and the intertragical notch, and so that a first surface of the housing is situated below a plane defined by the tragus, the antitragus and the antihelix.

In one embodiment, the earplug reduces a wide range of noise frequencies, from around 0 Hz up to 10 kHz and above. In one embodiment, the earplug reduces noise from a frequency of 12 Hz. Typically, the auditory threshold of humans varies from 16 to 20 Hz. In one embodiment the earplugs comprise both passive and active noise cancellation components. The earplugs may reduce 90 dB noise level down to 50 dB. For some frequencies the earplugs may reduce around 85 dB noise level down to 40 dB. For some frequencies the earplugs may reduce around 70 dB noise level down to 30 dB. For some frequencies, the reduction in noise level may be 36 dB. For some frequencies a noise of around 50 dB may be reduced to 0 dB or close to 0 dB, and may thus be cancelled practically entirely.

In one embodiment, the earplug small enough to fit into the user's ear entirely, so that leaning against a pillow or a headrest during sleep or rest is more comfortable and may be possible without the earplug dropping out of the ear e.g. during sleep. Such earplugs may be used inside a tight helmet such as a motorcycle helmet.

In one embodiment, the earplugs do not require an outside power source while in use, but operate on a battery. In one embodiment the battery together with the ANC circuit is configured to operate at least 25 hours, or at least 7 hours. Such an operation period is typically sufficient to last for a night sleep or a long-distance flight.

### BRIEF DESCRIPTION OF DRAWINGS

The accompanying drawings, which are included to provide a further understanding of earplug, and which constitute a part of this specification, illustrate embodiments of the earplug. Together with the description the drawings are meant to help to explain the principles of the earplug. The earplug is not limited to the specific embodiments illustrated in the drawings.

In the drawings:
**Figure 1** presents a simplified sectional view of an earplug according to the present disclosure.
**Figure 2** presents an axonometric projection of a pair of earplugs.
**Figures 3a** and **3b** present the earplug in two different axonometric projections.
**Figures 4a****-c** are further schematic presentations showing the dimensions of the earplug of a right ear.

### DETAILED DESCRIPTION OF THE EMBODIMENTS

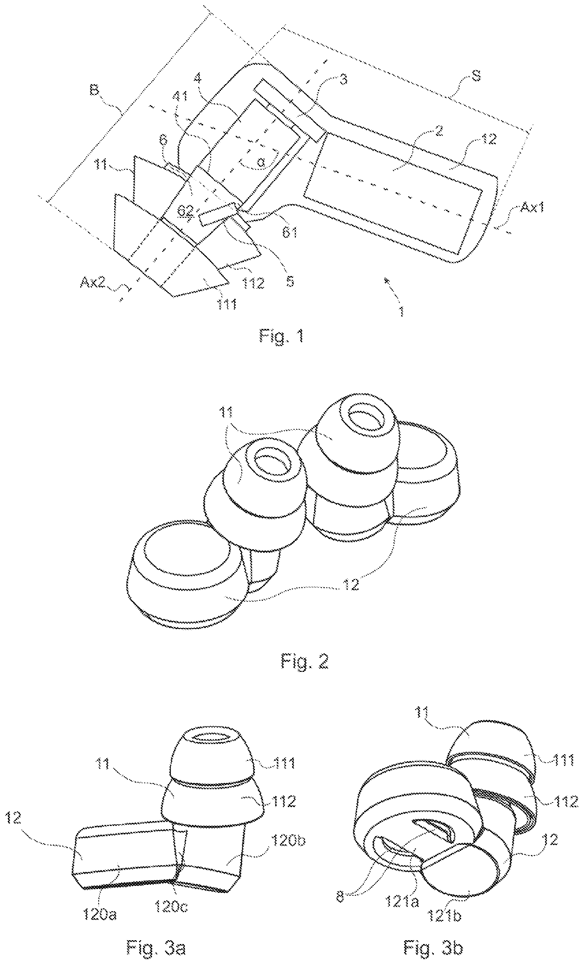

Figure 1 is a schematic sectional view of an earplug 1, in accordance with an example embodiment. It is understood that the earplug 1 as illustrated and hereinafter described is merely illustrative of an earplug that benefit from the embodiments of the disclosure and, therefore, should not be taken to limit the scope of the disclosure. In an embodiment, the earplug 1 may be adapted merely to reduce noise. In another embodiment, the earplug 1 may be adapted with audio listening capability.

A user would normally use a pair of earplugs 1, but for the sake of simplicity, the following description deals with one earplug 1 (for the right ear) only. Since a pair of earplugs is intended to be used in the left and right ear of the user, the earplugs 1 may be implemented as mirror images of each other. From the point of view of the functions, structure and design, the two earplugs need not differ from each other otherwise, as can be seen in Fig. 2.

In at least one example embodiment, the earplug 1 comprises a housing 12 and an audio cavity 6. Inside the housing 12 there may be arranged a battery 2, an active noise cancellation (ANC) circuit 3 for producing anti-noise, and a speaker 4 for emitting the anti-noise as a sound wave. The earplug 1 may further comprise at least one microphone 5 for measuring ambient noise and feeding the measured ambient noise to the ANC circuit 3. The at least one microphone 5 may be situated in the audio cavity 6, outside the audio cavity 6, and/or outside the housing 12. The audio cavity 6 is intended for guiding the sound wave from the speaker 4 out of the earplug 1. There may be several microphones 5 arranged within the audio cavity 6 or within the housing 12 or outside both of them. The battery 2 is intended for powering at least the ANC circuit 3.

The earplug 1 and the housing 12, viewed from one side, may have an L-shaped form which comprises a stem portion S along a first axis Ax1 and a bar portion B along a second axis Ax2. The speaker 4 may be arranged into the intersection of the first axis Ax1 and the second axis Ax2. Along the first axis Ax1 may be arranged at least the speaker 4 and the battery 2. Along the second axis Ax2 may be arranged at least the speaker 4 and the microphone 5.

In at least one example embodiment, the housing 12 may be understood to comprise a first cylindrical part 120a arranged along the first axis Ax1 so that the normal axis of the first cylindrical part 120a is substantially transverse to the first axis Ax1; and a second cylindrical part 120b whose axis is the second axis Ax2. The first cylindrical part 120a and the second cylindrical part 120b may be joined together at an angle with a connector 120c. In another example embodiment the first cylindrical part 120a and the second cylindrical part 120b may be joined together at an angle by shape, wherein the housing 12 may be made from one mould so that the first cylindrical part 120a and the second cylindrical part 120b form integral parts.

In an embodiment, the housing 12 may further comprise a first surface 121a, which, when the earplug is placed in a user's ear, may be seen as the top circular plane surface of the first cylindrical part 120a. When the earplug 1 is in the user's ear the first surface 121a is an outer surface that can be seen when the earplug is in place.

The first surface 121a may be substantially parallel to the first axis Ax1, and arranged opposite the audio cavity 6. In an embodiment, a second surface 122, which, when the earplug is used, may function as the bottom circular plane surface of the first cylindrical part 120a, may be arranged opposite the first surface 121a, substantially parallel to the first axis Ax1, and on the same side as the audio cavity 6.

In an embodiment, a third surface 121b can be seen on top of the ear canal when the earplug is in the user's ear. The third surface 121b is the top circular plane surface of the second cylindrical part 120b.

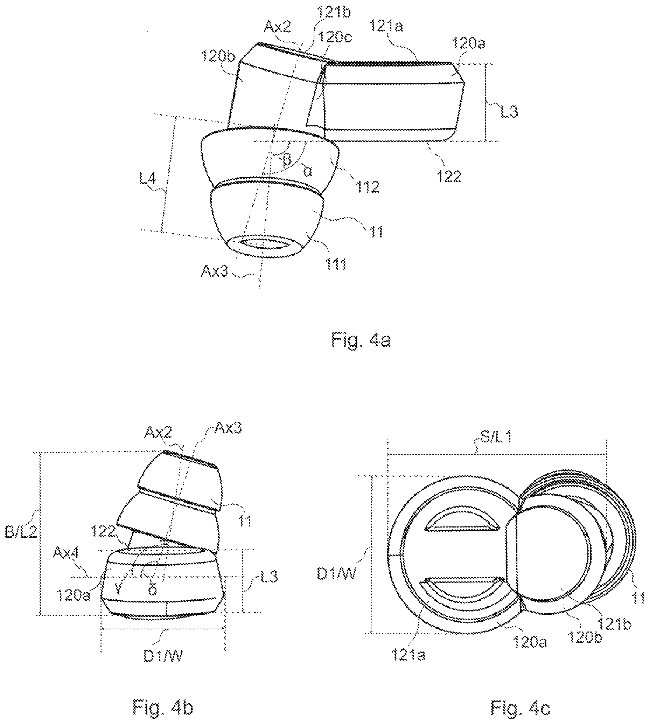

In at least one example embodiment, a first part 61 of the audio cavity 6 is situated within the housing 12, along the second axis Ax2. A purpose of the first part 61 may be to connect a sound-emitting face 41 of the speaker 4 to a second part 62 of the audio cavity 6, which second part 62 extends outwards from the housing 12, along a third axis Ax3 (see Fig. 4a). This outwards extending second part 62 of the audio cavity 6 may be covered with a sealing bud 11. The second axis Ax2 may be also the axis of the speaker 4.

A fourth axis Ax4 is shown in Fig. 4b. This is an axis perpendicular to the Axis Ax1, and parallel to the first surface 121a and the second surface 122.

In at least one embodiment, the sealing bud 11 functions as a passive noise reduction part 11 for physically blocking ambient noise. In an embodiment, the sealing bud 11 is removable by e.g. pulling, and can be placed back onto the housing 12 e.g. by pushing. In another embodiment the sealing bud 11 forms an integral part of the housing 12.

In an embodiment, the dimensions of the audio cavity 6 are chosen to have a diameter as large as possible, however, without causing discomfort to the user when the earplug 1 is in place. The dimensions, for example the diameter, of the audio cavity 6 may depend on the diameter of the speaker 4. The first part 61 and the second part 62 of the audio cavity 6 may also have other than a circular cross-sectional shape.

In at least one example embodiment, the diameter of the first part 61 of the audio cavity 6 inside the housing 12 may be for example 4,24 mm, and the diameter may vary from 2 to 7 mm. In some example embodiments, the diameter of the first part 61 may be 3 mm, or 5,5 mm or 6,25 mm. The diameter of the second part 62 of the audio cavity 6 may be for example 2,5 mm, and the diameter may vary from 2 to 3 mm. In some example embodiments, the diameter of the second part 62 may be 2,2 mm, or 2,65 mm, or 2,8 mm.

A higher level of noise attenuation may be achieved with a larger audio cavity diameter together with a small front cavity volume of the speaker 4. In at least some embodiments, the volume of the first part 61 and the second part 62 of the audio cavity 6 are kept small, because acoustic capacitance may increase with increasing volume.

A very narrow audio cavity 6 may increase acoustic resistance and inductance when utilising impedance analogy where pressure equals voltage, and volume velocity equals current. Thus, in at least some example embodiments, a designer would seek a balance between a suitable acoustic capacitance and high enough noise attenuation.

In at least some example embodiments, the earplug has such a shape and size that the connection between the microphone 5 and the user's eardrum is straight. Such a straight connection may minimise or at least reduce resistance, inductance and capacitance by arranging the microphone 5 and the eardrum essentially in the same acoustic space. Forming a common acoustic space between the microphone and the user's eardrum may enable the production of desired anti-noise up to the eardrum. By desired anti-noise is herein meant anti-noise of such frequency so as to attenuate disturbing noise.

In at least one example embodiment, the earplug 1 comprises at one end of the housing 12, along the first axis Ax1 a sealing bud 11. The sealing bud 11 may be detachably connectable to the casing of the second part 62 of the audio cavity 6. In an embodiment, the second part 62 of the audio cavity and the sealing bud 11 have a common axis Ax3 which is illustrated in Figure 4a.

In at least one example embodiment, the sealing bud 11 comprises a first conical flange part 111 and a second conical flange part 112 arranged on top of each other so that the first conical flange part 111 partly covers the second conical flange part 112. The second conical flange part 112 may be arranged to cover the second part 62 of the audio cavity 6 which is not situated within the housing 12 in order to achieve efficient acoustic attenuation of noise, sufficient sealing of the ear canal and comfortability for the user through a good fit in the ear canal. The double layer or double flange 111, 112 structure of the sealing bud 11 enables, in at least one example embodiment, the earplug 1 to be fitted tightly into the ear canal of the user so that the ear canal is effectively blocked off to enable attenuation of high frequency noise.

In at least one example embodiment, the sealing bud 11 may be made of silicone or other pliable, elastic and flexible material, such as memory foam or rubber. Such exemplary materials allow the sealing bud 11 to function as a passive noise reduction unit, and allow the insertion of the earplug 1 into the ear canal of a user. Such sealing bud materials may improve the conformation of the sealing bud 11 against the inner lining of the ear canal of a user to efficiently block out high frequency noise.

In at least one example embodiment, the earplug may be equipped (e.g. in the sales package) with sealing buds 11 of different sizes. Sealing buds of different sizes may be arranged to be sold separately as accessories. The user may select a sealing bud 11 in a size that best fits into his ear. The earplug may be arranged with sealing buds 11 of at least two different sizes (smaller and larger). Sealing buds 11 of different sizes may be of different colour or may have a letter (e.g. L for large, M for medium, S for small) to indicate size to the user.

By selecting a sealing bud 11 of suitable size the earplug 1 may, in at least one example embodiment, be inserted deep enough into the ear canal of the user's ear so as to avoid the outer surface 121a, 121b of the earplug (when in the ear) to extend further out of the ear than the tragus, antitragus and antihelix of the ear. This may not be possible for all users even with different sizes of sealing buds 11 due to different users having ears of different shape and size.

In one example embodiment, the length L4 (as illustrated in Figure 4a) of the smaller sealing bud 11, may be 9,05 mm, in the vertical direction, i.e. in the direction of the ear canal. In one example embodiment the length L4 of the smaller sealing bud 11 may vary between 8 and 10 mm (see Fig. 4a). In some example embodiments, the length L4 of the smaller sealing bud 11 may be 8,25 mm, or 8,7 mm, or 9,75 mm. In one example embodiment, the length L4 of the larger sealing bud 11 may be 10,82 mm, in the vertical direction, i.e. in the direction of the ear canal. In one example embodiment the length L4 of the larger sealing bud 11 may vary between 9 and 12 mm. In some example embodiments, the length L4 of the larger sealing bud 11 may be 9,5 mm, or 10,2 mm, or 11,45 mm. The earplug 1, its sales package or a container for storing one or several earplugs may be arranged with sealing buds of one, two or more different sizes.

In an example embodiment, within the audio cavity 6, the at least one microphone 5 may be arranged in electrical connection with the ANC circuit 3. In an embodiment, the microphone 5 may be arranged into the first part 61 of the audio cavity 6. In at least one example embodiment the microphone 5 is arranged in the earplug in relation to the speaker 4 so that when the earplug 1 is inserted into the user's outer ear, the microphone 5 is situated in front of the speaker 4, i.e. closer to or deeper inside the ear canal.

In an embodiment, the speaker 4 is in electrical connection with the ANC circuit 3. In at least one example embodiment, the ANC circuit 3 is placed within the housing 12 so that it is located behind the speaker 4 as seen from inside the ear canal, i.e. is further away from the ear canal than the speaker 4. In an embodiment, the battery 2 is arranged into the part of the housing 12 that, when the earplug 1 is in place as described above, rests inside the concha of the user's outer ear.

In an embodiment, as seen in a vertical direction of the ear canal, the battery 2 is arranged next to the speaker 4 so that in the vertical direction of the ear canal the battery 2 is located on a similar distance from the ear canal as the speaker 4.

In one embodiment, the active noise cancellation circuit 3 is configured to cancel noise at frequencies of 1 kHz or less. In another exemplary embodiment, that the active noise cancellation ANC circuit 3 is configured to cancel noise at frequencies of 2 kHz or less.

There are various known methods or topologies for implementing an ANC circuit. In at least one example embodiment, the ANC may employ a known feedback method or feedback topology for noise cancellation. With such an example ANC implementation, the microphone 5 may be arranged into the acoustic space in the earplug so as to locate towards the inner ear when in use, as illustrated in Figure 1, i.e. the microphone 5 is arranged within the audio cavity 6. The microphone 5 may be arranged along or in proximity of the second axis Ax2. In some example embodiments, the microphone 5 may be arranged in such a manner that at least a part of the microphone 5 intersects the second axis Ax2.

In an example embodiment, the ANC may employ a known feed-forward method or feed-forward topology for noise cancellation. With such an example ANC implementation, an additional microphone 5 is arranged into a space outside the ear and/or the earplug 1 when in use. In an example embodiment, "outside the ear and/or the earplug 1" means that the microphone 5 may be located into or below an audio opening arranged into the casing of the housing 12.

In an example embodiment, more than one microphone 5 may be employed with the feed-forward topology.

In an example embodiment, the ANC may employ a known combination or hybrid topology of the feedback and feed-forward methods/topologies. With such an example ANC implementation, at least two microphones 5 may be utilised, and they can be arranged either within the audio cavity 6, and/or outside the audio cavity 6, and/or outside the housing 12, as long as at least one microphone is arranged inside the audio cavity 6. In an example embodiment, "outside the housing 12" means that the microphone 5 may be located into or below an audio opening arranged into the casing of the housing 12.

With a known feedback method/topology implementation of the ANC circuit 3, the ANC circuit 3 may comprise an audio filter for filtering resonance peaks causing instability in the antinoise generation, thus improving the generation intensity of antinoise. An audio filter may comprise one or two or more, notch band stop filters for removing resonance derived from the speaker, the natural frequency of the ear canal and the so-called Helmholtz resonance of the audio cavity 6 of the earplug 1.

The audio filtering means may further comprise a lead-lag compensator for filtering infrasounds or for maintaining low frequency stability. Instability in the operation of the ANC manifests itself as oscillation, which may cause additional noises such as whistling or howling, humming, unpitched noise, or pops or clicks, disturbing to the user. The compensator may also be of the type lead, lag, PID or other with which a stable end result of infrasound filtering or low frequency stability maintaining can be achieved.

In at least one example embodiment, the battery 2 may be arranged within the housing 12 at least partly in the part of the stem portion S (of the L-shaped earplug) that does not intersect with the bar portion B (of the L-shaped earplug). In one example embodiment, at least part of the housing 12 on the stem portion S is a first cylindrically shaped part 120a, and the battery 2 is arranged within the first cylindrical part 120a in its entirety.

In an example embodiment, the housing 12 comprises charging sockets 8 arranged on the first surface 121a on the stem portion S, which is the upper surface of the earplug 1 as illustrated in Fig. 4a, i.e. when the L-shaped earplug is in a position where the stem portion S is in a horizontal position and the bar portion L is in a vertical position and facing downwards from the stem portion S.

In an embodiment, the battery 2 is rechargeable through the charging sockets 8. Such a placement of charging sockets 8 or charging pins 8 helps in avoiding skin contact with the charging sockets or pins 8 while the earplug 1 is in use and in place inside the outer ear, as the charging sockets 8 face outwards away from the outer ear.

In an example embodiment, the charging sockets 8 enable recharging the battery 2 with e.g. a separate external charger. In an embodiment, the charging sockets 8 are so formed that the earplug 1 can be easily positioned correctly onto charging spikes of an external charger. In an example embodiment, a cylindrically shaped Li-Ion battery 2 may be used. Li-Ion batteries exist of the suitable size and shape as to fit inside e.g. a housing 12 as the one illustrated in Fig. 1 with the exemplary dimensions provided herein, and that have enough energy to provide a sufficient operational period for the earplug 1. In an example embodiment, a sufficient operational period corresponds to a typical night's sleep, 7-9 hours, or to intercontinental flights of 10-14 hours.

In an embodiment, the operation time of the battery may be a minimum of 2 hours and may last even 50 hours in situations where the earplug 1 remains totally unmoved, i.e. if the user of the earplug 1 does not move his head. In an example embodiment and example use situation, the operation time may be 10 - 25 hours when the earplug 1 is in place and the user rests his head against a pillow or a headrest. In an example use situation, where the earplug 1 is subjected to constant movement, i.e. where the user keeps moving his head constantly or regularly, the operation time may be 7 hours.

In an embodiment, the external charger may also be utilised as a container for one or two earplugs 1 wherein charging plugs are arranged inside the container, and a charger may be connected to the container. In an example embodiment, the earplug 1 comprises a sensor for automatically activating, i.e. switching on the earplug and its ANC circuit and function, as soon as it is removed from its container. Examples of such a sensor are an ambient light sensor (ALS), a magnet (that may be provided in either or both the earplug and container), acceleration sensor. In an example embodiment, the earplug 1 comprises a sensor for automatically activating, i.e. switching on the earplug and its ANC circuit and function, as soon as it is placed in the ear if the user. Examples of such sensors are a proximity sensor and a camera, or another sensor that is capable of e.g. measuring the pulse of a human by sensing blood flow and thus sensing proximity of the earplug to the ear. Such a sensor may be arranged within or close to the sealing buds 11 (see e.g. Fig. 1) or at the surface 122 (see e.g. Fig. 4a).

In an example embodiment, the earplug 1 comprises an induction charging circuit. In such an embodiment no charging sockets or pins 8 are needed. The charger can in this case be an induction charger, such an induction charging pad onto which the earplug 1 is placed for charging the battery 2.

An example embodiment of the design of the earplug 1 is depicted in more detail in figures 1, 3a-b and 4a-c.

In an example embodiment, the housing 12 of the earplug 1 is formed of the first cylindrical part 120a and the second cylindrical part 120b. These two may be joined together at a specific point of their circumference at an angle α with the wedge-shaped connecting part 120c.

As can be seen in Fig. 1 and Fig. 4a, the angle α is, in the illustrated example embodiment, the inner angle between 1) the second axis Ax2 of the second cylindrical part 120b as well as the speaker 4, and 2) the first axis Ax1, which is also the plane defined by the second surface 122 of the housing 12. In an example embodiment, the angle α may vary from 85 to 120°. In an example embodiment, the angle α is 107,94°. In some other example embodiments, the angle α may be 85,25°, or 89,85°, or 93,5°, or 97,75°, or 102°, or 115,55°.

Further, in an example embodiment the second cylindrical part 120b is angled (angle β) in relation to the first cylindrical part 120a in another direction, as can be seen in Fig. 4a. The angle β is the inner angle between 1) the third axis Ax3 of the second part 62 of the audio cavity 6, which is also the axis of the sealing bud 11, and 2) the first axis Ax1, which is also the plane defined by the second surface 122 of the housing 2. In an example embodiment, the angle β may vary from 85 to 110°. In an example embodiment, the angle β is 92,56°. In some other example embodiments, the angle β may be 87,25°, or 95°, or 97,55°, or 103°.

Seen from another projection (Fig. 4b) there is also an angle γ between 1) the second axis Ax2 which is the axis of the second cylindrical part 120b as well as the speaker 4, and 2) the fourth axis Ax4 which defines a plane parallel to the first surface 120a and the second surface 122. The angle γ defines the inclination of the second cylindrical part 120b in relation to the first cylindrical part 120a. In an example embodiment, the angle γ may vary from 90 to 125°. In an example embodiment, the angle γ is 99,35°. In some other example embodiments, the angle γ may be 92,5°, or 96°, or 102,75°, or 116,25°, or 119,7°.

Furthermore, there is also an angle δ between 1) the third axis Ax3 which is the axis of the sealing bud 11 as well as the second part 62 of the audio cavity 6, and 2) the fourth axis Ax4 or the second surface 122. The angle δ defines the inclination of the sealing bud 11, i.e. the passive noise-cancelling part of the earplug 1, in relation to the first cylindrical part 120a. In an example embodiment, the angle δ may vary from 90 to 125°. In an example embodiment, the angle δ is 107,63°. In some other example embodiments, the angle δ may be 92,5°, or 97°, or 101,75°, or 112,25°, or 118,5°.

As a result of the selected inclined relationships between the different parts (11, 120b, 120a) of the earplug 1, the earplug 1 may have an anatomically correct design, meaning that is suitable and comfortable in use. As the second part 62 of the audio cavity 6 (as well as the sealing bud 11) is angled in relation to the second cylindrical part 120b from which it is arranged to protrude. In an example embodiment, the angles γ and δ may be selected or chosen so as to enable comfortable fit into the concha of the outer ear of the user, when the sealing bud 11 is in place in the ear canal. In an example embodiment, having such an angle β in the earplug 1 allows the earplug to be placed within the ear so as to provide room for earplug within the concha and intertragical notch, as well as to the tragus.

In Fig. 4a-c, dimensions of an example embodiment of the earplug 1 can be seen in more detail, in several different angles of view for the sake of clarity. The exemplary dimensions represent the dimensions of the earplug 1 so that when it is correctly placed inside the user's outer ear, the sealing bud 11 is inserted into the ear canal as far as it goes to ensure tight fit and efficient blocking off of the ear canal. The exemplary earplug 1 depicted in Fig. 3a-c is intended for the right ear.

In an example embodiment, the thickness L3 of the earplug 1, i.e. the thickness of the earplug 1 on the part of the stem portion S that does not intersect with the bar portion B, or the thickness of the first cylindrical part 120a, may be for 7,85 mm. In other example embodiments, the thickness L3 may vary from 5 to 9 mm (Figs. 4a, b). In some example embodiments, the thickness L3 may be 6,25 mm, or 7 mm, or 8,55 mm.

In an example embodiment, the width W of the earplug 1 is the diameter D1 of the first cylindrical part 120a, i.e. the width of the earplug 1 on the part of the stem portion S that does not intersect with the bar portion B. In an example embodiment, the width W is 15,75 mm. In other example embodiments, the width W may vary from 13 to 16 mm (Figs. 4b, c). In some example embodiments, the width W may be 13,5 mm, or 14,25 mm, or 15 mm.

In an example embodiment, the length L1 of the earplug is the length of the stem portion S, i.e. the measurement from the furthermost point on the perimeter of the first cylindrical part 120a to the furthermost point on the perimeter of the second cylindrical part 120b (Fig. 4c). In an example embodiment, the length L1 is 21,75 mm. In other example embodiments, the length L1 may vary from 17 to 25 mm. In some example embodiments, the length L1 may be 17,25 mm, or 19,55 cm, or 24,0 mm.

In an example embodiment, the length L2 of the bar portion B is the measurement from the first surface 121a of the second cylindrical part 120b to the outermost point of the sealing bud 11. In an example embodiment, this length L2 includes the dimensions of the housing 12 and the sealing bud 11 along the second axis Ax2. In an example embodiment, the length L2 is 19,79 mm, in case the user has selected a smaller sealing bud 11. In case a larger sealing bud 11 has been selected, the length L2 is in an example embodiment 21,56 mm. In other example embodiments, the length L2 may vary from 15 to 25 mm. In some example embodiments, the length L2 may be 16,55 mm, or 18,25 mm, or 21,05 mm, or 22 mm, or 23,75 mm.

In an example embodiment, the edges of the two cylindrical parts 120a, 120b may be rounded to ensure a comfortable fit inside the outer ear of the user. In an example embodiment, the edges between the first surface 121a of the housing 12 and the surface of the two cylindrical parts 120a, 120b may be bevelled. Such exemplary rounded or bevelled design may be beneficial in fitting the earplug 1 tightly but comfortably inside the outer ear, and may enable the earplug 1 to be inserted in its entirety into even a small ear, even when the structure of the user's ear is challenging (i.e. the crux helix is pronounced or situated low) so that when the earplug 1 is in the ear, the outer surfaces 121a, 121b remains within the borders of the ear created by the tragus, antitragus and the antihelix. The curved design of the outer contours of the housing 12 in an example embodiment allows the earplug 1 to be fitted against the antitragus of the outer ear.

In an example embodiment, the earplug 1 is inserted into place by pushing it into the outer ear and twisting it into place. In an example use situation, the earplug 1 is intended to be placed into the ear so that the bar portion B is placed in direction of and at least partly inside the user's ear canal, and so that the stem portion S extends from the ear canal to the concha. In an example embodiment, when the earplug 1 is properly in place inside the user's outer ear, the parts of the earplug 1 are situated as follows:
- the passive noise reduction part is inserted into the ear canal by pushing it tightly into a place where the sealing bud 11 effectively closes off the ear canal,
- the second cylindrical part 120b extends outwards from the ear canal to the intertragical notch of the outer ear,
- the first cylindrical part 120a nests comfortably inside the concha of the outer ear, and
- the first and third surfaces 121a, 121b end up situated below a plane defined by the tragus, the antitragus and the antihelix of the outer ear.

In this exemplary placement, the earplug 1 is situated completely inside the outer ear, i.e. no parts of the earplug 1 protrude outward or past the level defined by the tragus, the antitragus and the antihelix. This allows the user may comfortably rest his head and ear against a pillow or a headrest or similar without discomfort or pressure to the ear or head from the earplug 1 against the headrest. Furthermore, such an exemplary placement ensures that the earplug 1 cannot easily fall out from the outer ear.

The above embodiments are to be understood as illustrative examples of the earplug. Further embodiments of the earplug can be conceived. It is to be understood that any feature described herein in relation to any one embodiment may be used alone, or in combination with other features described, and may also be used in combination with one or more features of any other of the embodiments, or any combination of any other of the embodiments. The scope of protection is defined by the accompanying claims.

## Claims

1. A wireless noise-cancelling earplug (1) comprising a housing (12) within which at least an active noise cancellation ANC circuit (3) for producing anti-noise, a speaker (4) for emitting the anti-noise as a sound wave , and a battery (2) for powering at least the ANC circuit (3) are arranged; the earplug (1) further comprising an audio cavity (6) for guiding the sound wave from the speaker (4) out of the earplug (1), at least one microphone (5) for measuring ambient noise and feeding the measured ambient noise to the ANC circuit (3), and a passive noise reduction unit (11) for blocking ambient noise; the earplug (1) and the housing (12) as viewed from one side is L-shaped comprising a stem portion (S) of the L-shaped housing (12) along a first axis (Ax1) and bar portion (B) of the L-shaped housing (12) along a second axis (Ax2), an inner angle (α) between the first axis (Ax1) and the second axis (Ax2) of the L-shaped housing being 85 to 120 degrees, wherein the stem portion (S) has a length (L1) of 25 mm or less; and
the bar portion (B) has a length (L2) of 23 mm or less, wherein a thickness L3 of the earplug on the part of the stem portion (S) that does not intersect with the bar portion (B) is 5 to 9 mm; and
the at least one microphone (5) is arranged inside the audio cavity (6).

2. The earplug (1) according to claim 1, **characterised in that** the speaker (4) is arranged at AUXILIARY REQUEST 7_CLEAN
the intersection of the first axis (Ax1) and the second axis (Ax2); and the housing (12) comprises
along the first axis (Ax1) at least the speaker (4) and the battery (2); and
along the second axis (Ax2) at least the speaker (4) and the at least one microphone (5).

3. The earplug (1) according to claim 1 or 2, **characterised in that** the earplug (1) comprises at one end of the housing (12) along the second axis (Ax2) a sealing bud (11), wherein the length (L2) of the bar portion (B) includes the dimension of the housing (12) and the sealing bud (11) along the second axis (Ax2).

4. The earplug (1) according to any of claims 1 - 3, **characterised in that** the audio cavity (6) comprises a first part (61) arranged inside the housing (12) along the second axis (Ax2), and a second part (62) arranged to protrude from the housing (12) along a third axis (Ax3), so that an inner angle (β) between the first axis (Ax1) and the third axis (Ax3) is 85 - 110 degrees.

5. The earplug (1) according to any of the claims 1 - 4, **characterised in that** the housing (12) comprises a first cylindrical part (120a) arranged along the first axis (Ax1) so that the normal axis of the first cylindrical part (120a) is substantially perpendicular to the first axis (Ax1); a second cylindrical part (120b) whose axis is the second axis (Ax2); a first surface (121a) which is the top circular plane surface of the first cylindrical part (120a) and which is parallel to the first axis (Ax1) and opposite the audio cavity (6); and a second surface (122), which is the bottom circular plane surface of the first cylindrical part (120a) and which is parallel to the first axis (Ax1) and on the same side as the audio cavity (6).

6. The earplug (1) according to claim 5, **characterized in that** the battery (2) is arranged within the housing (12) inside the first cylindrical part (120a).

7. The earplug (1) according to claim 5 or 6, **characterised in that** at least the active noise cancellation ANC circuit (3), the speaker (4) and the first part (61) of the audio cavity (6) are arranged within the second cylindrical part (120b).

8. The earplug (1) according to any of claims 5 - 7, **characterised in that** the width (W) of the first cylindrical part (120a) is 13 - 16 mm.

9. The earplug (1) according to any of claims 5 - 8, **characterised in that** the housing (12) comprises charging sockets (8) arranged on a first surface (121a) of the housing (12), which is the top circular plane surface of the first cylindrical part (120a) and which is parallel to the first axis (Ax1) and opposite the audio cavity (6), on the stem portion (S).

10. The earplug (1) according to any one of claims 1 - 9, **characterised in that** the active noise cancellation ANC circuit (3) is configured to cancel noise at frequencies of 1 kHz or less.

11. The earplug (1) according to any of claims 1 - 10, **characterised in that** in use the earplug (1) is intended to be placed into the ear so that the bar portion (B) is placed in direction of and at least partly inside the ear canal, and the stem portion (S) extends from the ear canal to the concha.

12. The earplug (1) according to any of claims 5 - 11, **characterised in that** in use the earplug (1) is intended to fit in its entirety within a space inside the outer ear so that the passive noise reduction part (11) fits into the outermost part of the ear canal and the housing (12) fits into a space comprising the concha and the intertragical notch, and so that a first surface (121a), which is the top circular plane surface of the first cylindrical part (120a) and which is parallel to the first axis (Ax1) and opposite the audio cavity (6), and a third surface (121b) of the housing (12) are situated below a plane defined by the tragus, the antitragus and the antihelix.

## Patentansprüche

1. Drahtloser störgeräuschunterdrückender Ohrstöpsel (1), umfassend ein Gehäuse (12), in dem zumindest eine aktive Störgeräuschunterdrückungs-ANC-Schaltung (3) zum Erzeugen von Anti-Störgeräusch, ein Lautsprecher (4) zum Aussenden des Anti-Störgeräuschs als eine Schallwelle, und eine Batterie (2) zur Stromversorgung zumindest der ANC-Schaltung (3) angeordnet sind; wobei der Ohrstöpsel (1) ferner einen Audiohohlraum (6) zum Leiten der Schallwelle vom Lautsprecher (4) aus dem Ohrstöpsel (1) heraus, zumindest ein Mikrofon (5) zum Messen von Umgebungsstörgeräusch und zum Einspeisen des gemessenen Umgebungsstörgeräuschs in die ANC-Schaltung (3) umfasst, und eine passive Störgeräuschreduktionseinheit (11) zum Blockieren von Umgebungsstörgeräusch; wobei der Ohrstöpsel (1) und das Gehäuse (12), betrachtet von einer Seite aus, L-förmig sind, umfassend einen Schaftabschnitt (S) des L-förmigen Gehäuses (12) entlang einer ersten Achse (Ax1) und einen Stegabschnitt (B) des L-förmigen Gehäuses (12) entlang einer zweiten Achse (Ax2), wobei ein Innenwinkel (α) zwischen der ersten Achse (Ax1) und der zweiten Achse (Ax2) des L-förmigen Gehäuses 85 bis 120 Grad beträgt, wobei der Schaftabschnitt (S) eine Länge (L1) von 25 mm oder weniger aufweist; und
der Stegabschnitt (B) eine Länge (L2) von 23 mm oder weniger aufweist,
wobei eine Dicke L3 des Ohrstöpsels an dem Teil des Schaftabschnitts (S), der sich mit dem Stegabschnitt (B) nicht schneidet, 5 bis 9 mm beträgt; und
das zumindest eine Mikrophon (5) im Inneren des Audiohohlraums (6) angeordnet ist.

2. Ohrstöpsel (1) nach Anspruch 1, **dadurch gekennzeichnet, dass** der Lautsprecher (4) am Schnittpunkt der ersten Achse (Ax1) und der zweiten Achse (Ax2) angeordnet ist; und das Gehäuse (12) entlang der ersten Achse (Ax1) zumindest den Lautsprecher (4) und die Batterie (2); und
entlang der zweiten Achse (Ax2) zumindest den Lautsprecher (4) und das zumindest eine Mikrofon (5) umfasst.

3. Ohrstöpsel (1) nach Anspruch 1 oder 2, **dadurch gekennzeichnet, dass** der Ohrstöpsel (1) an einem Ende des Gehäuses (12) entlang der zweiten Achse (Ax2) einen Abdämmungsknopf (11) aufweist, wobei die Länge (L2) des Stegabschnitts (B) die Abmessung des Gehäuses (12) und des Abdämmungsknopfs (11) entlang der zweiten Achse (Ax2) umfasst.

4. Ohrstöpsel (1) nach einem der Ansprüche 1-3, **dadurch gekennzeichnet, dass** der Audiohohlraum (6) einen ersten Teil (61), der im Inneren des Gehäuses (12) entlang der zweiten Achse (Ax2) angeordnet ist, und einen zweiten Teil (62), der angeordnet ist, so dass er aus dem Gehäuse (12) entlang einer dritten Achse (Ax3) herausragt, so dass ein Innenwinkel (β) zwischen der ersten Achse (Ax1) und der dritten Achse (Ax3) 85-110 Grad beträgt, umfasst.

5. Ohrstöpsel (1) nach einem der Ansprüche 1-4, **dadurch gekennzeichnet, dass** das Gehäuse (12) einen ersten zylindrischen Teil (120a), der entlang der ersten Achse (Ax1) angeordnet ist, so dass die normale Achse des ersten zylindrischen Teils (120a) im Wesentlichen senkrecht zur ersten Achse (Ax1) ist; einen zweiten zylindrischen Teil (120b), dessen Achse die zweite Achse (Ax2) ist; eine erste Oberfläche (121a), die die obere kreisförmige ebene Oberfläche des ersten zylindrischen Teils (120a) ist und die parallel zur ersten Achse (Ax1) und gegenüberliegend zum Audiohohlraum (6) ist; und eine zweite Oberfläche (122), die die untere kreisförmige ebene Oberfläche des ersten zylindrischen Teils (120a) ist und die parallel zur ersten Achse (Ax1) ist und auf der gleichen Seite ist wie der Audiohohlraum (6), umfasst.

6. Ohrstöpsel (1) nach Anspruch 5, **dadurch gekennzeichnet, dass** die Batterie (2) innerhalb des Gehäuses (12) innerhalb des ersten zylindrischen Teils (120a) angeordnet ist.

7. Ohrstöpsel (1) nach Anspruch 5 oder 6, **dadurch gekennzeichnet, dass** zumindest die aktive Störgeräuschunterdrückungs-ANC-Schaltung (3), der Lautsprecher (4) und der erste Teil (61) des Audiohohlraums (6) innerhalb des zweiten zylindrischen Teils (120b) angeordnet sind.

8. Ohrstöpsel (1) nach einem der Ansprüche 5-7,
**dadurch gekennzeichnet, dass** die Breite (W) des ersten zylindrischen Teils (120a) 13-16 mm beträgt.

9. Ohrstöpsel (1) nach einem der Ansprüche 5-8,
**dadurch gekennzeichnet, dass** das Gehäuse (12) Ladebuchsen (8), die auf einer ersten Oberfläche (121a) des Gehäuses (12), die die obere kreisförmige ebene Oberfläche des ersten zylindrischen Teils (120a) ist und die parallel zur ersten Achse (Ax1) und gegenüber dem Audiohohlraum (6) liegt, auf dem Schaftabschnitt (S) angeordnet sind.

10. Ohrstöpsel (1) nach einem der Ansprüche 1-9, **dadurch gekennzeichnet, dass** die aktive Störgeräuschunterdrückungs-ANC-Schaltung (3) eingerichtet ist, Störgeräusch bei Frequenzen von 1 kHz oder weniger zu unterdrücken.

11. Ohrstöpsel (1) nach einem der Ansprüche 1-10, **dadurch gekennzeichnet, dass** der Ohrstöpsel (1) bei Gebrauch so in das Ohr eingesetzt werden soll, dass der Stegabschnitt (B) in Richtung des und zumindest teilweise innerhalb des Gehörgangs platziert ist, und der Schaftabschnitt (S) sich vom Gehörgang bis zur Ohrmuschel erstreckt.

12. Ohrstöpsel (1) nach einem der Ansprüche 5-11, **dadurch gekennzeichnet, dass** der Ohrstöpsel (1) bei Gebrauch in seiner Gesamtheit in einen Raum innerhalb des Außenohrs passen soll, so dass der passive Störgeräuschreduzierungsteil (11) in den äußersten Teil des Gehörgangs passt und das Gehäuse (12) in einen Raum passt, der die Ohrmuschel und die intertragische Kerbe umfasst, und so, dass eine erste Oberfläche (121a), die die obere kreisförmige ebene Oberfläche des ersten zylindrischen Teils (120a) ist und die parallel zur ersten Achse (Ax1) und gegenüberliegend zum Audiohohlraum (6) ist, und eine dritte Oberfläche (121b) des Gehäuses (12) unterhalb einer Ebene, die durch den Tragus, den Antitragus und die Antihelix definiert ist, angeordnet sind.

## Revendications

1. Oreillette antibruit sans fil (1) comprenant un boîtier (12) à l'intérieur duquel au moins un circuit antibruit actif ANC (3) pour produire un son antagoniste, un haut-parleur (4) pour émettre le son antagoniste en tant qu'une onde sonore, et une batterie (2) pour alimenter au moins le circuit ANC (3) sont agencés; l'oreillette (1) comprenant en outre une cavité audio (6) pour guider l'onde sonore depuis le haut-parleur (4) hors de l'oreillette (1), au moins un microphone (5) pour mesurer un bruit ambiant et fournir le bruit ambiant mesuré au circuit ANC (3), et une unité de réduction de bruit passive (11) pour bloquer un bruit ambiant; l'oreillette (1) et le boîtier (12), en vue depuis un côté, sont en forme de L comprenant une portion de tige (S) du boîtier en forme de L (12) le long d'un premier axe (Ax1) et une portion de barre (B) du boîtier en forme de L (12) le long d'un deuxième axe (Ax2), un angle intérieur (α) entre le premier axe (Ax1) et le deuxième axe (Ax2) du boîtier en forme de L étant de 85 à 120 degrés, dans laquelle la portion de tige (S) a une longueur (L1) inférieure ou égale à 25 mm; et
la portion de barre (B) a une longueur (L2) inférieure ou égale à 23 mm, dans laquelle une épaisseur L3 de l'oreillette sur la partie de la portion de tige (S) qui n'est pas en intersection avec la portion de barre (B) est de 5 à 9 mm ; et
l'au moins un microphone (5) est agencé à l'intérieur de la cavité audio (6).

2. Oreillette (1) selon la revendication 1, **caractérisée en ce que** le haut-parleur (4) est agencé à l'intersection du premier axe (Ax1) et du deuxième axe (Ax2) ; et le boîtier (12) comprend
le long du premier axe (Ax1), au moins le haut-parleur (4) et la batterie (2) ; et
le long du deuxième axe (Ax2), au moins le haut-parleur (4) et l'au moins un microphone (5).

3. Oreillette (1) selon la revendication 1 ou 2, **caractérisée en ce que** l'oreillette (1) comprend, à une extrémité du boîtier (12) le long du deuxième axe (Ax2), un cache de scellement (11), dans laquelle la longueur (L2) de la portion de barre (B) inclut la dimension du boîtier (12) et le cache de scellement (11) le long du deuxième axe (Ax2).

4. Oreillette (1) selon l'une quelconque des revendications 1 à 3, **caractérisée en ce que** la cavité audio (6) comprend une première partie (61) agencée à l'intérieur du boîtier (12) le long du deuxième axe (Ax2), et une seconde partie (62) agencée pour faire saillie depuis le boîtier (12) le long d'un troisième axe (Ax3), de sorte qu'un angle intérieur (β) entre le premier axe (Ax1) et le troisième axe (Ax3) soit de 85 à 110 degrés.

5. Oreillette (1) selon l'une quelconque des revendications 1 à 4, **caractérisée en ce que** le boîtier (12) comprend une première partie cylindrique (120a) agencée le long du premier axe (Ax1) de sorte que l'axe normal de la première partie cylindrique (120a) soit sensiblement perpendiculaire au premier axe (Ax1) ; une seconde partie cylindrique (120b) dont l'axe est le deuxième axe (Ax2) ; une première surface (121a) qui est la surface de plan circulaire de sommet de la première partie cylindrique (120a) et qui est parallèle au premier axe (Ax1) et à l'opposé de la cavité audio (6) ; et une deuxième surface (122), qui est la surface de plan circulaire de fond de la première partie cylindrique (120a) et qui est parallèle au premier axe (Ax1) et du même côté que la cavité audio (6).

6. Oreillette (1) selon la revendication 5, **caractérisée en ce que** la batterie (2) est agencée à l'intérieur du boîtier (12) dans la première partie cylindrique (120a).

7. Oreillette (1) selon la revendication 5 ou 6, **caractérisée en ce qu'**au moins le circuit antibruit actif ANC (3), le haut-parleur (4) et la première partie (61) de la cavité audio (6) sont agencés à l'intérieur de la seconde partie cylindrique (120b).

8. Oreillette (1) selon l'une quelconque des revendications 5 à 7, **caractérisée en ce que** la largeur (W) de la première partie cylindrique (120a) est de 13 à 16 mm.

9. Oreillette (1) selon l'une quelconque des revendications 5 à 8, **caractérisée en ce que** le boîtier (12) comprend des prises de chargement (8) agencées sur une première surface (121a) du boîtier (12), qui est la surface de plan circulaire de sommet de la première partie cylindrique (120a) et qui est parallèle au premier axe (Ax1) et à l'opposé de la cavité audio (6), sur la portion de tige (S).

10. Oreillette (1) selon l'une quelconque des revendications 1 à 9, **caractérisée en ce que** le circuit antibruit actif ANC (3) est configuré pour l'annulation de bruit à des fréquences inférieures ou égales à 1 kHz.

11. Oreillette (1) selon l'une quelconque des revendications 1 à 10, **caractérisée en ce que**, en utilisation, l'oreillette (1) est destinée à être placée dans l'oreille de sorte que la portion de barre (B) soit placée dans la direction du conduit auditif et au moins partiellement à l'intérieur de celui-ci, et la portion de tige (S) s'étend depuis le conduit auditif jusqu'à la conque.

12. Oreillette (1) selon l'une quelconque des revendications 5 à 11, **caractérisée en ce que**, en utilisation, l'oreillette (1) est destinée à s'ajuster dans son intégralité à l'intérieur d'un espace dans l'oreille externe de sorte que la partie de réduction de bruit passive (11) s'ajuste dans la partie la plus extérieure du conduit auditif et le boîtier (12) s'ajuste dans un espace comprenant la conque et l'échancrure de la conque, et de sorte qu'une première surface (121a), qui est la surface de plan circulaire de sommet de la première partie cylindrique (120a) et qui est parallèle au premier axe (Ax1) et à l'opposé de la cavité audio (6), et une troisième surface (121b) du boîtier (12) soient situées au-dessous d'un plan défini par le tragus, l'antitragus et l'anthélix.
